# FASCICULE DE BREVET EUROPEEN

(11) **EP 1 345 594 B1**
(45) Date de publication et mention de la délivrance du brevet: **24.01.2007**
(21) Numéro de dépôt: 01989654.7
(22) Date de dépôt: 21.12.2001
(51) Int. Cl.: A61K 9/16, A61K 9/20, A61K 31/55

(54) **COMPOSITION PHARMACEUTIQUE SOLIDE THERMOFORMABLE POUR LA LIBERATION CONTROLEE D'IVABRADINE**
WÄRMEFORMBARE FESTE PHARMAZEUTISCHE ZUSAMMENSETZUNG FÜR DIE KONTROLLIERTE FREIGABE VON IVABRADIN
THERMOFORMABLE SOLID PHARMACEUTICAL COMPOSITION FOR CONTROLLED RELEASE OF IVABRADINE

(30) Priorité: 26.12.2000 FR 0017015
(43) Date de publication de la demande: 24.09.2003
(73) Titulaire: Les Laboratoires Servier, 92415 Courbevoie Cedex (FR)
(72) Inventeur: WUTHRICH, Patrick, F-45000 Orléans (FR); ROLLAND, Hervé, F-45160 Olivet (FR); BRIAULT, Gilles, 91670 Angerville (FR); PICHON, Gérald, F-45100 Orléans (FR); THARRAULT, François, F-45000 Orléans (FR)
(86) Numéro de dépôt international: PCT/FR2001/004134
(87) Numéro de publication internationale: WO 2002/051387

(56) Documents cités:
- EP-A- 0 544 144
- FR-A- 2 681 862
- US-A- 5 965 161

## Description

La présente invention concerne une nouvelle composition pharmaceutique solide, pour la libération contrôlée d'Ivabradine, obtenue par thermoformage à chaud d'un mélange à base de polymère(s) appartenant à la famille des polyméthacrylates.

De nombreuses compositions pharmaceutiques destinées à la libération contrôlée de principes actifs pharmaceutiques ont été proposées et réalisées, pour leur administration par voie orale, buccale, sublinguale, oculaire, rectale, vaginale et/ou parentérale. Ces nouvelles compositions avaient essentiellement pour objectifs :
- de réduire la fréquence d'administration des médicaments,
- d'obtenir des taux relativement constants de principe actif dans le milieu ou au site biologique visé,
- d'obtenir des profils de libération en corrélation avec l'activité pharmacologique des médicaments.
   Pour contrôler cette libération, le principe le plus communément employé est d'incorporer le ou les principes actifs avec des excipients, le plus souvent de nature polymérique, dans des matrices.

Quelles que soient les compositions matricielles envisagées, leur obtention se heurte à des problèmes spécifiques de fabrication :
- procédé de fabrication complexe et en plusieurs étapes,
- stabilité du principe actif au cours du procédé de fabrication et vis-à-vis des excipients utilisés,
- modulation de la vitesse de libération, du ou des principes actifs, délicate, souvent variable dans le temps et dépendante par exemple de la granulométrie des lots de polymères avec les procédés de compression,
- procédé de fabrication permettant l'obtention d'une forme pharmaceutique essentiellement adaptée à une seule voie d'administration,
- reproductibilité des lots du fait de la multiplication des étapes.

L'Ivabradine, composé de formule : 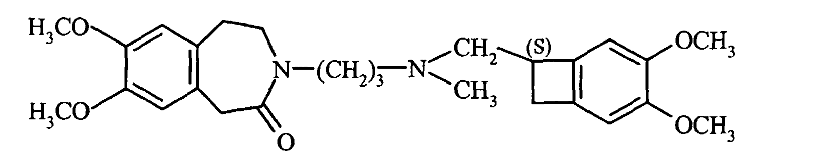 est un régulateur sino-atrial, exclusivement bradycardisant utile pour le traitement de l'angor stable.

FR 2681862 décrit des nouvelles (benzocycloalkyl) alkylamines, par exemple l'Ivabradine, et leur procédé de préparation.

L'objet de la présente invention est une alternative permettant de surmonter les difficultés d'ordre général précédemment exposées pour l'obtention de compositions pharmaceutiques solides permettant la libération contrôlée d'Ivabradine et de ses sels pharmaceutiquement acceptables par l'utilisation des techniques de thermoformage. Elle permet notamment de réduire le nombre d'étapes pour la production de formes galéniques finalisées, limitant ainsi les problèmes de reproductibilité et le coût économique, et assurant aussi un gain de temps et d'espace au sein d'une chaîne de production.

Plus particulièrement, l'invention vise une nouvelle application des polyméthacrylates à la réalisation desdites compositions pharmaceutiques solides sans addition de plastifiant et sans addition d'agents modulant la libération du ou des principes actifs. L'invention, telle que mise en oeuvre par la Demanderesse permet ainsi de restreindre le nombre de produits intervenant au sein d'une formulation galénique, limitant ainsi les problèmes de stockage et d'approvisionnement, ainsi que les problèmes liés à la gestion de l'environnement.

Le thermoformage à chaud concerne notamment les techniques de l'extrusion, de la co-extrusion, de l'injection et de la co-injection. Ces différentes techniques sont bien connues dans le domaine de la plasturgie et ont été largement utilisées dans le secteur de l'automobile ou de l'emballage.

De part leurs caractéristiques, et les propriétés physicochimiques des polymères utilisables pour le thermoformage, ces techniques, et notamment l'extrusion simple, sont de plus en plus appliquées au domaine de la mise en forme des principes actifs.

Différents brevets décrivent ainsi des compositions pharmaceutiques à libération contrôlée qui sont obtenues par extrusion d'un mélange comportant au moins, un principe actif, un ou plusieurs polymères extrudables et pharmaceutiquement acceptables, un plastifiant et/ou un retardant, ce dernier composé permettant de moduler la libération du principe actif.

C'est le cas plus particulièrement de la demande de brevet WO 96/14058 qui revendique une composition pharmaceutique incluant notamment comme agent thérapeutique un opioïde qui est dispersé dans une matrice réalisée par extrusion. La matrice à extruder comprend ainsi un principe actif, un matériel hydrophobe qui peut être fondu, tel qu'un alkyle cellulose ou un polymère acrylique ou méthacrylique, et un matériel hydrophobe tel qu'un acide gras ou un alcool gras. Ce dernier composé joue le rôle de retardant et permet de ralentir et contrôler la libération dudit principe actif. Afin de diminuer la température d'extrusion, un plastifiant est ajouté au mélange.

Le brevet US 5,102,668 décrit une composition pharmaceutique à libération contrôlée, indépendante du pH, ladite composition étant obtenue par extrusion humide de polymères tels que les polyméthacrylates, lesdits polymères étant hydrophiles à faible pH et hydrophobes à fort pH. Le polyméthacrylate utilisé préférentiellement est de l'Eudragit® E100. Les extrudats ainsi obtenus doivent ensuite subir une étape de sphéronisation, puis de façon avantageuse, ils sont recouverts d'un film de polymère constitué d'Eudragit® NE 30 D. L'association entre le polymère constituant l'extrudat et le polymère constituant le film de revêtement permet de résoudre le problème technique particulier de cette invention qui est le contrôle de la libération du principe actif en fonction du pH du milieu de dissolution.

Parmi l'art antérieur, on peut également citer le brevet DE 41 38 513 qui présente un procédé de préparation d'une composition pharmaceutique à libération contrôlée, par extrusion en continu d'un mélange comprenant au moins un principe actif, un polyméthacrylate, et un polymère de N-vinylpyrrolidone et/ou d'hydroxyalkyl (méthyl)cellulose. Ces derniers composés sont utilisés en tant que plastifiant et jouent un rôle dans la régulation de la libération contrôlée du principe actif.

Dans l'article *Pharm. Res.* 1996, 13 (5), 804-808, il est également décrit l'extrusion à chaud d'Eudragit^{®} E100 additionné de plastifiant, au moins 12 % de triéthylcitrate, pour l'obtention de films permettant la libération contrôlée de principes actifs.

De même, les revues *J. Cont. Rel.* 1995, 36, 243-250 et *Drug Dev. Ind. Pharm.* 1994, 20, 1323-1339 rapportent l'utilisation de l'Eudragit^{®} RS PM additionné de plastifiant (triacetine) pour l'obtention de granules par extrusion à chaud. La cinétique de libération du principe actif est rapide et les granules ne libèrent pas la totalité du principe actif. Les températures d'extrusion se situent entre 130°C et 140°C.

Ces différents documents décrivent ainsi l'application de la technique de l'extrusion simple pour l'obtention de nouvelles compositions pharmaceutiques. Les techniques de l'injection et de la co-injection ont été beaucoup moins étudiées et elles concernent principalement des compositions pharmaceutiques solides dont la matrice est à base de dérivés cellulosés, d'amidon ou de polyéthylène glycol.

Enfin, concernant la technique de la co-extrusion, la demande de brevet FR 2 766 088 décrit un procédé pour la production d'un article à partir duquel peuvent être fabriqués des dispositifs à libération contrôlée, ledit procédé consistant à effectuer une co-extrusion de polymère et de principe actif, le polymère utilisé étant de préférence un composé organosilicié capable de se réticuler en la présence ou en l'absence d'agent de réticulation.

La présente invention permet, d'une façon simple et économique, d'obtenir directement une composition pharmaceutique solide, à libération contrôlée, par simple mélange d'Ivabradine ou d'un de ses sels pharmaceutiquement acceptables et de polymère(s) ayant des propriétés plastiques et étant pharmaceutiquement acceptables, sans ajout de plastifiant ou de retardant, ledit mélange étant thermoformé. Le contrôle de la libération du principe actif contenu dans ladite composition est obtenu uniquement grâce à un choix judicieux du ou des polymères plastiques utilisés, et de leur quantité relative par rapport à celle du principe actif. Les compositions pharmaceutiques selon l'invention, outre le fait qu'elles soient nouvelles, permettent d'obtenir des formes galéniques aisément adaptables à l'Ivabradine et à ses sels pharmaceutiquement acceptables et à son meilleur mode d'administration, et assurent une libération contrôlée et reproductible de ce dernier.

L'un des objets de l'invention était de mettre au point une composition pharmaceutique solide, à libération contrôlée, contenant un simple mélange d'Ivabradine ou de ses sels pharmaceutiquement acceptables, et de polymère(s) ayant des propriétés plastiques, le(s)dit(s) polymère(s) étant constitué(s) par le groupe des polyméthacrylates, sans ajout de plastifiant, et/ou de retardant, et sans utilisation de solvant.

D'une façon surprenante, les compositions pharmaceutiques solides de la Demanderesse, de part leur constitution spécifique, peuvent aussi bien être soumises à la technique de l'extrusion, de la co-extrusion qu'à celle de l'injection ou de la co-injection. La mise en oeuvre de ces techniques permet d'aboutir à l'obtention de matrices sous des formes de taille et de géométrie appropriées aux diverses voies d'administration telles que notamment la voie orale, buccale, sublinguale, oculaire, vaginale, rectale, et parentérale. Cet avantage des compositions pharmaceutiques de ladite invention permet d'envisager la fabrication, à partir de la même matière première, de la formulation galénique la mieux adaptée à la fois à l'Ivabradine et à ses sels pharmaceutiquement acceptables incorporé dans ladite composition, et à sa meilleure voie d'administration et de la population devant utiliser ces formulations.

Enfin, un des objets de l'invention était d'obtenir une composition pharmaceutique solide où il était possible de moduler simplement la libération de l'Ivabradine, par simple adaptation des quantités de celui-ci et de polymères plastiques utilisés.

Plus spécifiquement, la présente invention concerne une composition pharmaceutique solide à libération contrôlée, administrable notamment par voie orale, contenant un mélange thermoformable, d'Ivabradine et de ses sels pharmaceutiquement acceptables et d'un ou plusieurs polymères choisis parmi le groupe des polyméthacrylates, la libération contrôlée de l'Ivabradine étant uniquement assurée par la nature chimique, la quantité du ou des polyméthacrylates utilisés, et la technique mise en oeuvre pour la fabrication de ladite composition.

Dans les compositions pharmaceutiques selon l'invention, l'Ivabradine se trouve préférentiellement sous la forme de chlorhydrate.

Par composition pharmaceutique à libération contrôlée, on comprend une libération d'Ivabradine sur une durée de quelques minutes correspondant à une libération immédiate, à une durée de plus de 20 heures correspondant à une libération prolongée, ladite libération pouvant s'effectuer de façon décalée dans le temps après absorption de la composition. Dans le cas de composition pharmaceutique à libération décalée, le temps de latence correspondant au temps entre l'absorption de ladite composition et la libération du principe actif, peut être d'une durée de 30 minutes à 8 heures, la libération du principe actif pouvant ensuite être une libération immédiate ou une libération prolongée telle que définis précédemment. Dans le cadre de l'invention, il est également possible d'obtenir des compositions pharmaceutiques présentant une combinaison des profils de libération telle qu'une libération immédiate d'une partie du principe actif suivie d'une ou plusieurs libérations décalées.

Par polyméthacrylate, on entend un copolymère d'acide méthacrylique correspondant à un copolymère totalement polymérisé d'acide méthacrylique et d'ester acrylique ou méthacrylique. Ces polyméthacrylates sont couramment appelés Eudragit^{®}, et peuvent se présenter sous la forme d'une poudre ou de granulés.

Par mélange thermoformable, on entend un mélange apte à subir une transformation par l'effet combiné de la chaleur et des forces de cisaillement d'une vis sans fin comme les techniques de l'extrusion, de la co-extrusion, de l'injection, et de la co-injection.

Parmi les différents Eudragit^{®} commercialisés, ceux utilisés préférentiellement dans le cadre de l'invention sont les Eudragit^{®} RL et RS qui se réfèrent à des copolymères de méthacrylate ammonium consistant en des copolymères totalement polymérisés d'acide acrylique et d'ester d'acide méthacrylique contenant une faible quantité de groupes ammonium quaternaire.

Ces Eudragit^{®} répondent à la formule générale (I) : 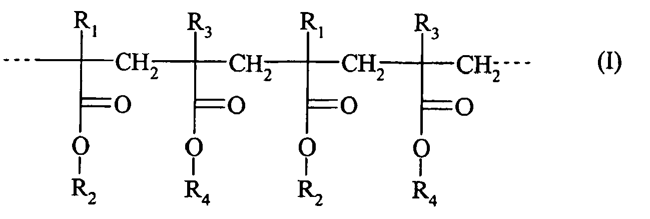 dans laquelle :
R₁ représente un atome d'hydrogène ou un groupement méthyle,
R₂ représente un groupement méthyle ou éthyle,
R₃ représente un groupement méthyle, et R₄ représente un groupement

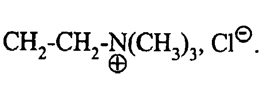

D'une façon particulièrement avantageuse, les Eudragit^{®} utilisés au sein du mélange thermoformable de l'invention, sont les Eudragit^{®} RLPO et/ou RSPO correspondant à des poly(éthyle acrylate, méthyle méthacrylate, chlorure de triméthylaminoéthyle méthacrylate) dans des proportions relatives respectives de 1:2:0,2 et 1:2:0,1.

Selon une autre variante avantageuse de l'invention, le mélange thermoformable de l'invention peut contenir de l'Eudragit^{®} de type E. Ce polymère correspond à un poly(butylméthacrylate, (2-diméthylaminoéthyl)méthacrylate, méthylméthacrylate) dans des proportions relatives 1:2:1. L'Eudragit^{®} de type E peut être utilisé comme seul polymère polyméthacrylate, au sein du mélange thermoformable, ou en association avec de l'Eudragit^{®} RLPO et/ou RSPO.

Parmi les Eudragit^{®} de type E, on peut citer notamment l'Eudragit^{®} E100 dont la particularité est d'être soluble à des pH inférieurs à 5, permettant une libération rapide du principe actif au niveau gastrique. De ce fait, l'utilisation d'Eudragit^{®} de type E, et plus particulièrement de type E100, est particulièrement bien adaptée à l'obtention de compositions pharmaceutiques solides à libération immédiate, administrables par voie orale.

Selon une troisième variante de l'invention, le mélange thermoformable de l'invention peut contenir de l'Eudragit^{®} de type L100, L100-55 et/ou S100. L'Eudragit^{®} L100 correspond à un poly(méthacrylique acide, méthylméthacrylate) dans des proportions relatives 1:1. L'Eudragit^{®} L100-55 correspond à un poly(méthacrylique acide, éthylacrylate) dans des proportions relatives 1:1. L'Eudragit^{®} S100 correspond à un poly(méthacrylique acide, méthylméthacrylate) dans des proportions relatives 1:2. Ces catégories d'Eudragit^{®} peuvent être utilisées comme seul polymère polyméthacrylate au sein du mélange thermoformable ou en association avec un ou plusieurs des autres types d'Eudragit^{®} cités précédemment. Ces polyméthacrylates sont solubles à des pH supérieurs à 5,5, permettant ainsi une libération de principe actif au niveau intestinal et/ou colonique. L'utilisation de cesdits Eudragit^{®} est particulièrement intéressante pour l'obtention de compositions pharmaceutiques solides gastrorésistantes, à libération contrôlée.

Les compositions pharmaceutiques ainsi obtenues dans le cadre de l'invention permettent d'obtenir, de façon inattendue, une libération contrôlée d'Ivabradine sur une durée de quelques minutes à plus de 20 heures, celle-ci pouvant être linéaire, la constitution de la matrice, et la technique mise en oeuvre.

Ainsi, les compositions pharmaceutiques de l'invention sont obtenues par mélange d'Ivabradine ou de ses sels pharmaceutiquement acceptables et d'un ou plusieurs polymères polyméthacrylates, abaissement de la viscosité de ce mélange sous l'effet de la chaleur et des forces de cisaillement d'une vis sans fin à l'intérieur d'un fourreau, puis traitement de ce mélange fondu selon l'une des voies suivantes :
- soit expulsion de l'extrudeuse à travers un orifice calibré, de taille et de forme variable, le matériau obtenu étant ensuite découpé selon la taille finale souhaitée de la matrice. Ceci constitue la technique de l'extrusion simple,
- soit la première extrudeuse contenant ledit mélange, de viscosité réduite, décrit précédemment est associée à une seconde extrudeuse contenant un mélange comportant :
   * soit uniquement un ou plusieurs polyméthacrylate(s) pour le contrôle de la libération du principe actif à partir du compartiment central,
   * soit un ou plusieurs polyméthacrylate(s) en mélange avec un ou plusieurs principe(s) actif(s), identique(s) ou différent(s) de celui (ou ceux) contenu(s) dans le compartiment central,
   chaque extrudeuse fonctionnant en continu et alimentant le même orifice.
   Cet orifice permet le passage du mélange provenant de la première extrudeuse et assure la formation de la couche interne de la matrice finale, ainsi que le passage du mélange provenant de la seconde extrudeuse et assurent la formation de la couche externe de la matrice finale. L'extrudat ainsi obtenu est ensuite découpé selon la taille finale souhaitée, et peut éventuellement subir un moulage. Les extrémités de l'extrudat peuvent éventuellement être fermées par une technologie appropriée. Ceci constitue la technique de co-extrusion,
- soit injection sous pression, au sein d'une presse, dans des moules de forme et de volume parfaitement définis selon les caractéristiques géométriques souhaitées pour la matrice. Ceci constitue la technique de l'injection,
- soit la presse est équipée de plusieurs unités d'injection permettant l'injection dans un même moule, de façon séquentielle ou simultanées, d'au moins deux mélanges, identiques ou différents. La première unité d'injection injecte ledit mélange, décrit précédemment, celui-ci constituant la partie centrale, ou coeur, de la matrice. La deuxième unité d'injection injecte à la périphérie de la partie centrale, une couche externe d'un mélange comportant :
   * soit uniquement un ou plusieurs polyméthacrylate(s) pour le contrôle de la libération d'Ivabradine ou de ses sels d'addition,
   * soit un ou plusieurs polyméthacrylate(s) en mélange avec l'Ivabradine ou un de ses sels d'addition, identique(s) ou différent(s), de celui (ou ceux) contenu(s) dans la partie centrale.
Ceci constitue la technique de co-injection qui regroupe à la fois les techniques d'injection multimatières et d'injection "sandwich".

Selon la technique employée, il est donc possible d'obtenir dans le cadre de la présente invention des compositions pharmaceutiques solides, administrables notamment par voie orale, buccale, sublinguale, oculaire, rectale, vaginale ou parentérale, à libération contrôlée, de taille et de géométrie variées, monocouche ou multicouche, adaptées au mieux selon les profils de libération les plus adéquates pour l'Ivabradine.

Ces compositions pharmaceutiques peuvent être utilisées directement, sans autre opération technique de transformation, hormis leur conditionnement. Si on le souhaite, lesdites compositions pharmaceutiques peuvent toutefois subir une transformation par broyage ou par granulation pour une mise en gélule ou pour être comprimées, ou être soumise à un enrobage.

Les compositions pharmaceutiques de l'invention peuvent aussi contenir éventuellement des excipients, pharmacologiquement acceptables, choisis par exemple parmi le groupe des antioxydants, des aromatisants, des colorants, des conservateurs, des édulcorants, et des antiadhérents.

La température de thermoformage est comprise entre 60°C et 150°C. D'une façon préférentielle, la température est comprise entre 80 et 130°C.

Les exemples suivants illustrent l'invention.

### EXEMPLE A : Extrusion

Les compositions de cet exemple sont obtenues par la technique de l'extrusion. Elles sont réalisées avec le chlorhydrate d'Ivabradine et sont dosées à 10, 20 et 50 mg d'Ivabradine équivalent base.

Les compositions sont constituées de mélange contenant 10, 20 et 50 % d'Ivabradine et respectivement 90, 80 et 50 % de polyméthacrylates RLPO et RSPO, seul ou en mélange.

Cet exemple montre l'influence d'une part de la nature et du pourcentage des polyméthacrylates utilisés et d'autre part le pourcentage en Ivabradine, sur la cinétique de dissolution in vitro du principe actif.

La température d'extrusion pour les lots est comprise entre 100 et 110°C. L'extrusion est réalisée avec une filière de 4 mm de diamètre et la vitesse de vis est de 10 tours/minute.

**Tableau 1 : Composition des lots étudiés**

| ***N° de lot*** | ***Ivabradine (%)*** | ***Type d'Eudragit*^{®} *(%)*** |
|---|---|---|
| Lot 1 | 10 | RLPO (*90*) |
| Lot 2 | 10 | RSPO (*90*) |
| Lot 3 | 20 | RLPO (*80*) |
| Lot 4 | 50 | RLPO (*50*) |
| Lot 5 | 10 | RLPO/RSPO-(90/10) (*90*) |
| Lot 6 | 50 | RLPO/RSPO-(90/10) (*50*) |
| Lot 7 | 10 | RLPO/RSPO-(50/50) (*90*) |
| Lot 8 | 50 | RLPO/RSPO-(50/50) (*50*) |

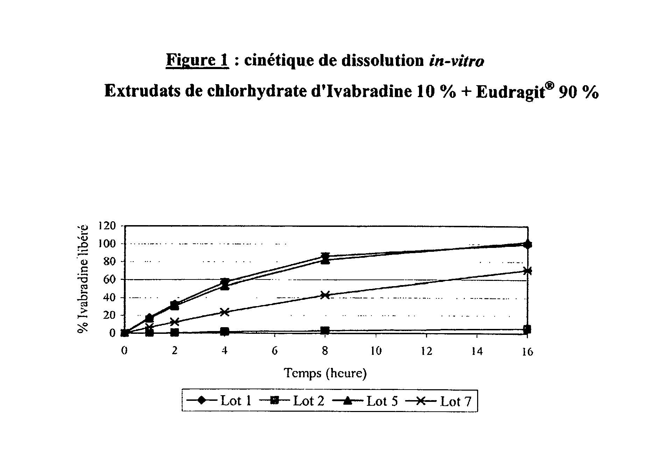

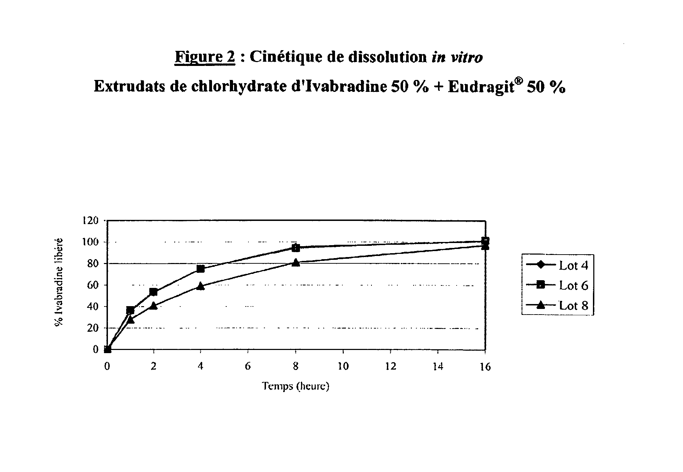

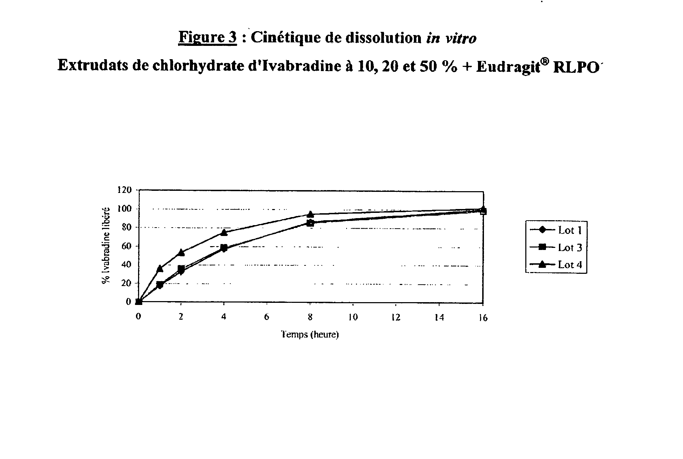

Les cinétiques de dissolution in vitro sont mesurées par dosage en chromatographie liquide haute performance (HLPC) d'extrudats d'environ 100 mg, correspondant à 10, 20 ou 50 mg d'Ivabradine équivalent base dans un milieu de dissolution tamponné de pH 6,8.
Les cinétiques de dissolution sont présentées en annexe dans les figures 1, 2 et 3.
Les résultats montrent que selon le type et le pourcentage d'Eudragit^{®} utilisé et selon le pourcentage en Ivabradine, la cinétique de libération du chlorhydrate d'Ivabradine peut être modulée en fonction du temps.

### EXEMPLE B : Injection

Les compositions de cet exemple sont obtenues par la technique de l'injection. Elles sont réalisées avec le chlorhydrate d'Ivabradine et sont constituées de mélange contenant 10 et 50 % d'Ivabradine et respectivement 90 et 50 % de polymétacrylates RLPO et RSPO, seul ou en mélange.

La température d'injection est comprise entre 115 et 125°C. Les formes injectées obtenues sont des cylindres de 2 mm d'épaisseur pour un diamètre de 6 mm de masse égale à 67 mg.

**Tableau 2 : Composition des lots étudiés**

| ***N° de lot*** | ***Ivabradine (%)*** | ***Type d'Eudragit*^{®} *(%)*** |
|---|---|---|
| Lot 10 | 10 | RLPO/RSPO-(80/20)- (*90*) |
| Lot 11 | 10 | RLPO (*90*) |
| Lot 12 | 50 | RLPO (*50*) |

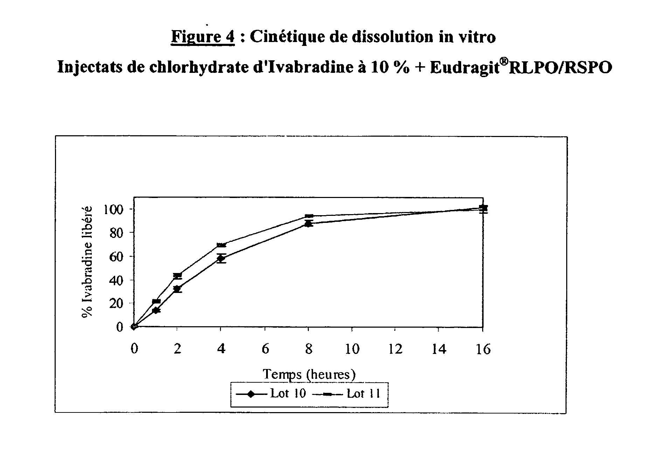

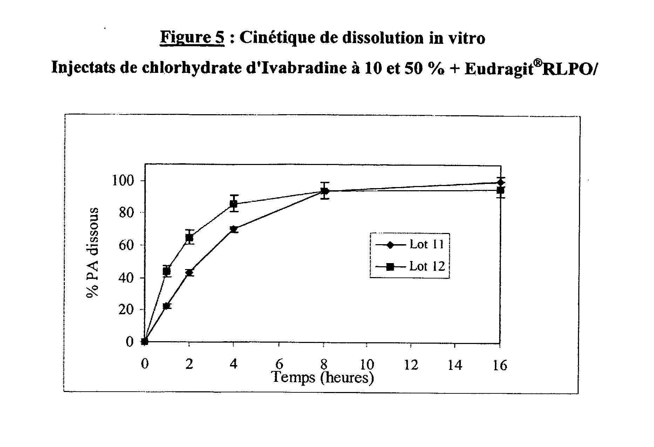

Les cinétiques de dissolution in vitro sont mesurées comme dans l'exemple A et sont présentées en annexe dans les figures 4 et 5. Les résultats montrent que la cinétique de dissolution est modulée en fonction du type et du pourcentage d'Eudragit^{®}, ainsi que de la quantité d'Ivabradine.

## Revendications

1. Composition pharmaceutique solide pour la libération contrôlée d'Ivabradine ou de ses sels pharmaceutiquement acceptables, **caractérisée en ce qu'**elle comporte un mélange thermoformable d'Ivabradine ou d'un de ses sels pharmaceutiquement acceptables et d'un ou plusieurs polymères choisis parmi le groupe des polyméthacrylates, la libération d'Ivabradine étant uniquement contrôlée par la nature du ou des polyméthacrylates utilisés, leur quantité relative par rapport à l'Ivabradine, et par la technique mise en oeuvre pour la fabrication de ladite composition.

2. Composition pharmaceutique solide à libération contrôlée selon la revendication 1 **caractérisée en ce que** le ou les polyméthacrylate(s) utilisé(s) dans le mélange thermoformable appartienne(nt) à la famille des copolymères de méthacrylate ammonium consistant en des copolymères totalement polymérisés d'acide acrylique et d'ester d'acide méthacrylique contenant une faible quantité de groupes ammonium quaternaire.

3. Composition pharmaceutique solide à libération contrôlée selon l'une quelconque des revendications 1 et 2 **caractérisée en ce que** le ou les polyméthacrylate(s) utlisé(s) dans le mélange thermoformable est (sont) des poly(ethyle acrylate, méthyle méthacrylate, chlorure de triméthylaminoéthyle méthacrylate) dans des proportions relatives de 1:2:0,2 et/ou 1:2:0,1.

4. Composition pharmaceutique solide à libération contrôlée selon l'une quelconque des revendications 1 à 3 **caractérisée en ce que** le mélange thermoformable comporte un poly(butylméthacrylate, (2-diméthylaminoéthyl)méthacrylate, méthylméthacrylate) dans des proportions relatives 1:2:1, seul ou en association avec un ou plusieurs des copolymères totalement polymérisés d'acide méthacrylique et d'ester acrylique ou méthacrylique cités précédemment.

5. Composition pharmaceutique solide à libération contrôlée selon l'une quelconque des revendications 1 à 4, **caractérisée en ce que** le mélange thermoformable comporte un poly(méthacrylique acide, méthylméthacrylate) dans des proportions relatives 1:1, un poly(méthacrylique acide, éthylacrylate) dans des proportions relatives 1:1 et/ou un poly(méthacrylique acide, méthylméthacrylate) dans des proportions relatives 1:2, seul(s) ou en association avec un ou plusieurs des copolymères totalement polymérisés d'acide méthacrylique et d'ester acrylique ou méthacrylique cités précédemment.

6. Composition pharmaceutique solide à libération contrôlée selon la revendication 1, **caractérisée en ce que** ladite composition est administrable selon l'une des voies choisies parmi orale, buccale, sublinguale, oculaire, vaginale, rectale et parentérale.

7. Composition pharmaceutique solide à libération contrôlée selon l'une quelconque des revendications 1 à 6, **caractérisée en ce que** ladite composition est administrable par voie orale.

8. Composition pharmaceutique solide à libération contrôlée selon la revendication 1 **caractérisée en ce que** la température de thermoformage du mélange est comprise entre 60°C et 150°C.

9. Composition pharmaceutique solide à libération contrôlée selon l'une quelconque des revendications 1 et 8, **caractérisée en ce que** la température de thermoformage du mélange est comprise entre 80°C et 130°C.

10. Composition pharmaceutique solide à libération contrôlée selon la revendication 1, **caractérisée en ce que** le mélange est thermoformé selon la technique de l'extrusion.

11. Composition pharmaceutique solide à libération contrôlée selon la revendication 1, **caractérisée en ce que** le mélange est thermoformé selon la technique de l'injection.

12. Composition pharmaceutique solide à libération contrôlée selon la revendication 1, **caractérisée en ce que** le mélange est thermoformé selon la technique de la co-extrusion, la couche interne de ladite composition étant alors constituée par ledit mélange et la couche externe de ladite composition étant constituée par un ou plusieurs polyméthacrylate(s), soit par un ou plusieurs polyméthacrylate(s) en mélange avec l'Ivabradine ou l'un de ses sels pharmaceutiquement acceptables.

13. Composition pharmaceutique solide à libération contrôlée selon la revendication 1, **caractérisée en ce que** le mélange est thermoformé selon la technique de la co-injection, la partie centrale de ladite composition étant alors constituée par ledit mélange et la couche externe de ladite composition étant constituée soit par un ou plusieurs polyméthacrylate(s), soit par un ou plusieurs polyméthacrylate(s) en mélange avec l'Ivabradine ou l'un de ses sels pharmaceutiquement acceptables.

14. Composition pharmaceutique solide à libération contrôlée selon la revendication 1, **caractérisée en ce qu'**elle comporte éventuellement un ou plusieurs excipients, pharmacologiquement acceptables, choisis parmi les antioxydants, les aromatisants, les colorants, les conservateurs, les édulcorants et les antiadhérents.

15. Composition pharmaceutique solide selon la revendication 1 **caractérisée en ce que** l'Ivabradine est sous forme de chlorhydrate.

## Claims

1. Solid pharmaceutical composition for the controlled release of ivabradine or a pharmaceutically acceptable salt thereof, **characterised in that** it comprises a thermoformable mixture of ivabradine or a pharmaceutically acceptable salt thereof and of one or more polymers selected from the group of the polymethacrylates, the release of the ivabradine being controlled solely by the nature of the polymethacrylate(s) used, by the amount thereof relative to the ivabradine and by the technique employed in the manufacture of the said composition.

2. Solid controlled-release pharmaceutical composition according to claim 1, **characterised in that** the polymethacrylate(s) used in the thermoformable mixture belong(s) to the family of copolymers of ammonium methacrylate that consist of fully polymerised copolymers of acrylic acid and of methacrylic acid ester having a small amount of quaternary ammonium groups.

3. Solid controlled-release pharmaceutical composition according to either claim 1 or claim 2, **characterised in that** the polymethacrylate(s) used in the thermoformable mixture is/are poly(ethyl acrylate, methyl methacrylate, trimethylaminoethyl methacrylate chloride)'s in the relative proportions of 1:2:0.2 and/or 1:2:0.1.

4. Solid controlled-release pharmaceutical composition according to any one of claims 1 to 3, **characterised in that** the thermoformable mixture comprises a poly(butyl methacrylate, (2-dimethylaminoethyl) methacrylate, methyl methacrylate) in the relative proportions of 1:2:1, alone or in association with one or more of the fully polymerised copolymers of methacrylic acid and of acrylic or methacrylic ester mentioned hereinbefore.

5. Solid controlled-release pharmaceutical composition according to any one of claims 1 to 4, **characterised in that** the thermoformable mixture comprises a poly(methacrylic acid, methyl methacrylate) in the relative proportions of 1:1, a poly(methacrylic acid, ethyl acrylate) in the relative proportions of 1:1 and/or a poly(methacrylic acid, methyl methacrylate) in the relative proportions of 1:2, alone or in association with one or more of the fully polymerised copolymers of methacrylic acid and of acrylic or methacrylic ester mentioned hereinbefore.

6. Solid controlled-release pharmaceutical composition according to claim 1, **characterised in that** the said composition is administrable by one of the routes selected from the oral, buccal, sublingual, ocular, vaginal, rectal and parenteral routes.

7. Solid controlled-release pharmaceutical composition according to any one of claims 1 to 6, **characterised in that** the said composition is administrable by the oral route.

8. Solid controlled-release pharmaceutical composition according to claim 1, **characterised in that** the temperature of thermoforming of the mixture is from 60°C to 150°C.

9. Solid controlled-release pharmaceutical composition according to either claim 1 or claim 8, **characterised in that** the temperature of thermoforming of the mixture is from 80°C to 130°C.

10. Solid controlled-release pharmaceutical composition according to claim 1, **characterised in that** the mixture is thermoformed according to the technique of extrusion.

11. Solid controlled-release pharmaceutical composition according to claim 1, **characterised in that** the mixture is thermoformed according to the technique of injection.

12. Solid controlled-release pharmaceutical composition according to claim 1, **characterised in that** the mixture is thermoformed according to the technique of co-extrusion, the inner layer of the said composition in this case being composed of the said mixture and the outer layer of the said composition being composed either of one or more polymethacrylate(s) or of one or more polymethacrylate(s) in admixture with ivabradine or a pharmaceutically acceptable salt thereof.

13. Solid controlled-release pharmaceutical composition according to claim 1, **characterised in that** the mixture is thermoformed according to the technique of co-injection, the central portion of the said composition in this case being composed of the said mixture and the outer layer of the said composition being composed either of one or more polymethacrylate(s) or of one or more polymethacrylate(s) in admixture with ivabradine or a pharmaceutically acceptable salt thereof.

14. Solid controlled-release pharmaceutical composition according to claim 1, **characterised in that** it optionally contains one or more pharmacologically acceptable excipients selected from anti-oxidants, flavourings, colourings, preservatives, sweeteners and anti-adherents.

15. Solid pharmaceutical composition according to claim 1, **characterised in that** the ivabradine is in the form of the hydrochloride.

## Patentansprüche

1. Feste pharmazeutische Zubereitung zur gesteuerten Freisetzung von Ivabradin oder seinen pharmazeutisch annehmbaren Salzen, **dadurch gekennzeichnet, daß** sie eine wärmeformbare Mischung aus Ivabradin oder einem seiner pharmazeutisch annehmbaren Salze und einem oder mehreren Polymeren ausgewählt aus der Gruppe der Polymethacrylate umfaßt, wobei die Freisetzung von Ivabradin ausschließlich durch die Art des oder der verwendeten Polymethacrylate, deren Menge bezogen auf Ivabradin und die für die Herstellung der genannten Zubereitung eingesetzten Technik gesteuert wird.

2. Feste pharmazeutische Zubereitung zur gesteuerten Freisetzung nach Anspruch 1, **dadurch gekennzeichnet, daß** das (die) in der wärmeformbaren Mischung verwendete(n) Polymethacrylat(e) der Familie der Copolymeren von Ammoniummethacrylat angehört (angehören), die aus vollständig polymerisierten Copolymeren aus Acrylsäure und Methacrylsäureester bestehen und eine geringe Menge von quaternären Ammoniumgruppen enthalten.

3. Feste pharmazeutische Zubereitung zur gesteuerten Freisetzung nach einem der Ansprüche 1 und 2, **dadurch gekennzeichnet, daß** das (die) in der wärmeformbaren Mischung verwendete(n) Polymethacrylat(e) Poly(Ethylacrylat, Methylmethacrylat. Trimethylaminoethylmethacrylatchlorid) in den relativen Verhältnissen 1:2:0,2 und/oder 1:2:0,1 ist (sind).

4. Feste pharmazeutische Zubereitung zur gesteuerten Freisetzung nach einem der Ansprüche 1 bis 3, **dadurch gekennzeichnet, daß** die wärmeformbare Mischung ein Poly(Butylmethacrylat, (2-Dimethylaminoethyl)-methacrylat, Methylmethacrylat) in den relativen Verhältnissen 1:2:1 allein oder in Kombination mit einem oder mehreren vollständig polymerisierten Copolymeren von Methacrylsäure und Acryl- oder Methacrylester, wie sie oben definiert sind, umfaßt.

5. Feste pharmazeutische Zubereitung zur gesteuerten Freisetzung nach einem der Ansprüche 1 bis 4, **dadurch gekennzeichnet, daß** die wärmeformbare Mischung ein Poly(Methacrylsäure, Methylmethacrylat) mit den relativen Verhältnissen 1:1, ein Poly(Methacrylsäure, Ethylacrylat) in den relativen Verhältnissen 1:1 und/oder ein Poly(Methaerylsäure, Methylmethacrylat) in den relativen Verhältnissen 1:2 allein oder in Kombination mit einem oder mehreren der oben genannten vollständig polymerisierten Copolymeren von Methacrylsäure und Acryl- oder Methacrylester umfaßt.

6. Feste pharmazeutische Zubereitung zur gesteuerten Freisetzung nach Anspruch 1, **dadurch gekennzeichnet, daß** sie auf oralem, bukkalem, sublingualem, okularem, vaginalem, rektalem und/oder parenteralem Wege verabreichbar ist.

7. Feste pharmazeutische Zubereitung mit gesteuerter Freisetzung nach einem der Ansprüche 1 bis 6, **dadurch gekennzeichnet, daß** sie auf oralem Wege verabreichbar ist.

8. Feste pharmazeutische Zubereitung mit gesteuerter Freisetzung nach Anspruch 1, **dadurch gekennzeichnet, daß** die Temperatur der Wärmeformung der Mischung zwischen 60°C und 150°C liegt.

9. Feste pharmazeutische Zubereitung mit gesteuerter Freisetzung nach einem der Ansprüche 1 und 8, **dadurch gekennzeichnet, daß** die Temperatur der Wärmeformung der Mischung zwischen 80°C und 130°C liegt.

10. Feste pharmazeutische Zubereitung zur gesteuerten Freisetzung nach Anspruch 1, **dadurch gekennzeichnet, daß** die Mischung durch die Extrusionstechnik wärmegeformt wird.

11. Feste pharmazeutische Zubereitung zur gesteuerten Freisetzung nach Anspruch 1, **dadurch gekennzeichnet, daß** die Mischung durch die Spritztechnik wärmegeformt wird.

12. Feste pharmazeutische Zubereitung zur gesteuerten Freisetzung nach Anspruch 1, **dadurch gekennzeichnet, daß** die Mischung durch die Coextrusionstechnik wärmegeformt wird, wobei die innere Schicht der Zubereitung aus der Mischung gebildet wird und die äußere Schicht der Zubereitung durch ein oder mehrere Polymethacrylat(e) oder durch ein oder mehrere Polymethacrylat(e) in Mischung mit Ivabradin oder einem seiner pharmazeutisch annehmbaren Salze gebildet wird.

13. Feste pharmazeutische Zubereitung zur gesteuerten. Freisetzung nach Anspruch 1, **dadurch gekennzeichnet, daß** die Mischung durch die Co-Spritztechnik wärmegeformt wird, wobei der zentrale Teil der Zubereitung durch die Mischung gebildet wird und die äußere Schicht der Zubereitung entweder durch ein oder mehrere Polymethacrylat(e) oder durch ein oder mehrere Polymethacrylat(e) in Mischung mit Ivabradin oder einem seiner pharmazeutisch annehmbaren Salze gebildet wird.

14. Feste pharmazeutische Zubereitung nach Anspruch 1, **dadurch gekennzeichnet, daß** sie gegebenenfalls einen oder mehrere pharmazeutisch annehmbare Hilfsstoffe enthält, ausgewählt aus Antioxidantien, Aromatisierungsmitteln. Farbstoffen, Konservierungsmitteln, Süßungsmitteln und Antihaftmitteln.

15. Feste pharmazeutische Zubereitung nach Anspruch 1, **dadurch gekennzeichnet, daß** Ivabradin in Form des Hydrochlorids vorliegt.
